# EUROPEAN PATENT APPLICATION

(11) **EP 0 688 577 A1**
(43) Date of publication of application: **27.12.1995**
(21) Application number: 94109832.9
(22) Date of filing: 24.06.1994
(51) Int. Cl.: A61N 1/362, A61N 1/39

(54) **Device for treating atrial tachyarrhythmia**

(71) Applicant: Pacesetter AB, S-171 95 Solna (SE)
(72) Inventor: Holmström, Nils, S-175 49 Järfälla (SE); Hagel, Pia, S-191 50 Sollentuna (SE); Carlsten, Johan, S-753 24 Uppsala (SE); Stroetmann, Brigitte, Dr.rer.nat., D-91080 Uttenreuth (DE)
(74) Representative: Lettström, Richard Wilhelm

(57) **Abstract**

A device for supraventricular heart therapy, whereby the device contains an arrhythmia detector (5) for detecting supraventricular arrhythmia and a nerve stimulator (910) for emitting pulses, in response to said detection, to a physiological representative (27) of the parasympathetic nervous system via an electrode system (23) whereby the electrode system's comprises stimulation means (24) devised to be placeable in an extracardiac position in the neck area of the physiological representative (27) of the parasympathetic nervous system and for activating this nervous system in direct contact there with this representative (27) or via an adjacent blood vessel.

## Description

The present invention relates generally to a device for treating atrial tachyarrhythmia according to the preambula of patent claim 1.

Supraventricular tachyarrhythmias, i.e. atrial fibrillation or atrial tachycardia, are, if not acutely life-menacing, disturbing to patients who experience them and can lead to complications. Atrial fibrillation, which may be chronic, increases the risk of degeneration of atrial musculature, blood clots and ventricular fibrillation. Atrial tachycardia also places an abnormal load on the heart, since the heart's pumping capability is disrupted when the atrioventricular node (A-V node) is unable to provide regular conduction of the atrium's electrical activity.

Conventional treatment of supraventricular tachycardia is with drugs, such as digitalis, or electroshocks which can be delivered both transcutaneously with an external defibrillator or using an implantable defibrillator whose pulse generator emits its defibrillation pulses over an electrode situated in the atrial area. Tachycardia-terminating pacemakers, as well as ablation procedures, can also be used for therapeutic purposes.

An implantable atrial defibrillator (the designation "cardioverter" is also used when modest levels of energy are delivered), whose pulse energy is on the order of 5 J, is described in US-A-4 572 191. Even if such an atrial pulse is at the lower limit of the level required to defibrillate ventricular fibrillation (5-40 J), its energy is still considerable in terms of trauma to the patient and the load imposed on the implantable energy source's capacity in frequently recurrent arrhythmias. Typically, atrial arrhythmias can recur often, and a reduction in the energy required to terminate an atrial arrhythmia and cause the heart to return to sinus rhythm would therefore be particularly desirable.

In therapy in the form of electrical nerve stimulation for treatment of other disorders than tachyarrhythmias in the heart, it has long been known that the energy required is very small compared to the aforementioned energy levels required in conventional electrical defibrillation.

Electrical nerve stimulation and the energy needed for it can be elucidated with a modern system for stimulation of the vagus nerve in treatment of epilepsy and are described in an article by Tarver et al.: "Clinical Experience with a Helical Bipolar Stimulating Lead", Pace, Vol. 15, October, Part II 1992. The system employs a pulse generator which is subcutaneously implanted in the upper left part of the thorax region and connected to a nerve-stimulating electrode arranged around the left branch of the vagus nerve in the neck area. During nerve stimulation, the generator emits 0-12 mA pulses with a pulse width of 130 to 1000 microseconds.

The vagus nerve is also of interest as regards the heart and the nervous system's action thereon, is also of interest, since this nerve, which comprises a plurality of branches, forms a part of the autonomic nerve system. The autonomic nerve system innervates the heart in the form of two sub-systems, the sympathetic and the parasympathetic. From the physiological point of view, these system are represented by a plurality of nerves or nerve strings (with attendant ganglia) in different locations. The terms "sympathetic nerve" and "vagus nerve" will often be used below, for the sake of simplicity, for the two sub-systems and attendant nerves/nerve strings, despite the actual complexity of innervation. Additional information will be provided in the following description when needed. Increased signal activity in the sympathetic nerve increases heart activity (heart rate and stroke volume), whereas increased signal activity in the vagus nerve reduces heart activity (heart rate). Activity in the sympathetic nerve and the vagus nerve normally balance each other so the heart maintains an appropriate rate at rest: about 70 beats/minute.

Termination of impending or established ventricular tachyarrhythmias by electrical stimulation of the vagus nerve, more particularly its endocardiac ends, has recently been proposed (Max Schaldach "Electrotherapy of the Heart", 1992, Springer Verlag, Heidelberg, pp. 210-214). Here, the vagus nerve is activated in response to the detection indicating that predefined conditions for activity in the sympathetic nervous system have been met. Activity in the sympathetic nervous system is measured via measurement of intraventricular impedance in the heart. This document also suggests, in general terms without proposing any concrete design, that supraventricular tachyarrhythmias might also be treatable by stimulation of the vagus nerve.

The object of the present invention is to achieve an effective device for treating supraventricular tachyarrhythmias by means of nerve stimulation.

The object of the invention is achieved with the features set forth in the independent patent claim, and advantageous embodiments of the invention are defined in the dependent claims.

In one such advantageous embodiment, the device includes a pacemaker for emitting ventricular stimulation pulses to prevent cardiac arrest and to maintain normal blood pressure.

According to the invention, a supraventricular cardiac arrhythmia is therefore treated by delivering the pulses to a representative of the parasympathetic nervous system via a stimulation means, arranged in an extracardiac position in the neck area, which in direct contact with this representative or via an adjacent blood vessel activates this nervous system. Such stimulation from a stimulation means placeable in the neck area is advantageous since implantation is simple. When implantation is through the blood vessel, an additional advantage is that local surgical trauma is avoided in the specific stimulation position.

The invention will now be described in greater detail with reference to an embodiment as disclosed in the attached drawings, of the device, according to the invention, for heart therapy as applied in the above-described AICD defibrillator system. For illustrative - not restrictive - reasons, the device according to the invention will henceforth be designated in this description as a "nerve-stimulating heart defibrillator" or a "nerve-heart defibrillator" whose task is to terminate fibrillation in the heart. It is to be understood that also other tachyarrhythmias, such as impending but as yet unestablished fibrillation treated with ATP or cardioversion according to conventional technics, can be treated and that the designation "nerve-heart defibrillator" in this context is a term only employed for explanatory purposes. Even if the nerve-heart stimulator is explained and described in conjunction with a AICD-type defibrillator system, it is further understood that the nerve-heart stimulator can be employed independently without all the parts in the described defibrillator system.

FIG. 1 is a block diagram of a defibrillator system with the nerve-heart defibrillator according to the invention.

FIG. 2 is an example of a vagus nerve stimulator in the nerve-heart defibrillator.

FIG. 3 shows illustrative examples of patterns of vagus nerve stimulation pulses.

FIG. 4 shows an example of the constructive principles of a nerve electrode.

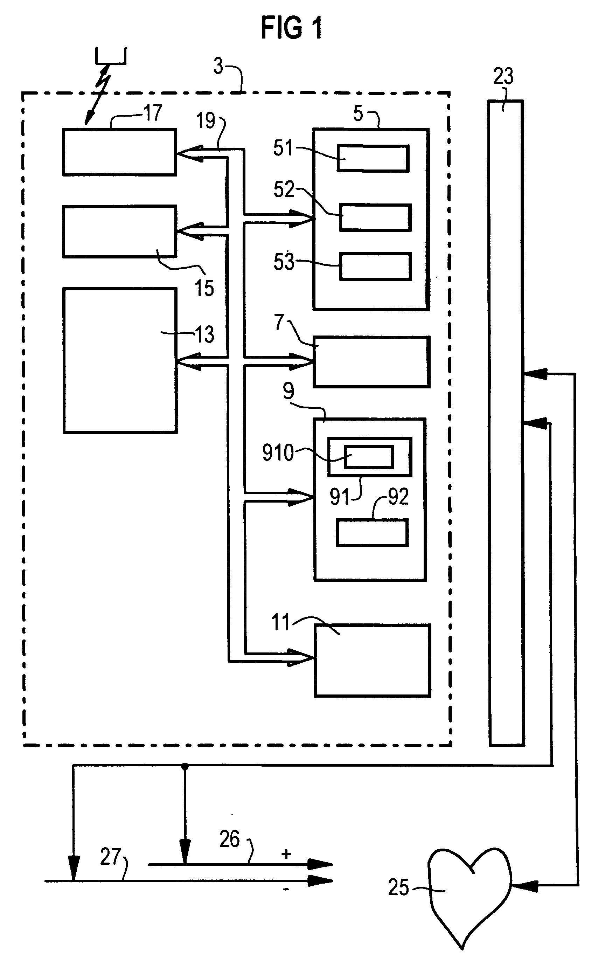

Referring to the block diagram in FIG. 1 there is shown an example of a defibrillation system using the nerve-heart defibrillator according to the invention, in which 1 designates a defibrillator implant in general. The implant 1 has an enclosure which could consist of e.g. a titanium capsule 3. The implant 1 comprises a detection block 5, a pacemaker block 7, which can emit stimulation pulses to the heart in both bradycardia and tachycardia, a block for nerve-heart defibrillation 9, a block for electrical defibrillation 11, a control unit 13, a diagnosis block 15 and a telemetry block 17, whereby the different blocks/units in the implant 1 communicate internally across a databus 19.

The implant 1 communicates with an external programmer 21 via the telemetry block 17, whereby communications primarily comprise the transmission of programming data to the implant 1 and transmission of diagnostic data, e.g. on different events in the heart, sensor signals and ECG signals, from the diagnostic block 15.

The implant 1 is connected to a heart 25 via a system 23 of electrode leads with attendant electrodes for emitting pacing as well as defibrillation pulses (including even pulses with somewhat less energy than the level required for defibrillation, e.g. cardioversion pulses) to the heart 25 and for picking up signals indicative of the heart's condition. It should be noted that FIG. 1 is only schematic, and the signals designating the heart's condition also encompass sensor signals from the sensing of heart-related physiological variables elsewhere in the body, e.g. hemodynamics (pressure/flow) in the vascular system. A blood pressure-sensing body could e.g. be arranged in the patient's neck area around a blood vessel (neck artery or vein), whereby the sensing body could consist of a ring-shaped (possibly suturable) holder device and a sensing device on the inside of the holder device in the form of a pressure-sensitive airbag.

The implant 1 is also in connection with the sympathetic nerve 26 (a plus sign designates an activating effect on heart activity) and the vagus nerve 27 (a minus sign designates an inhibitory effect on heart activity) via the system 23 of electrodes and electrode leads in order to emit nerve-stimulating pulses to the vagus nerve 27 and blocking current to the sympathetic nerve 26 and for picking up heart-related nerve signals therefrom.

The defibrillator implant 1 accordingly includes in addition to the nerve-heart defibrillator block 9 which is to be described e.g. the functions found in a modern defibrillator (AICD) of the kind noted above. Thus, the heart's condition is monitored in block 5 in an IECG-monitoring device 51 and in a sensor signal-monitoring device 52 (for e.g. hemodynamics). Heart-related nerve signals are also monitored in the detection block 5 in a nerve signal-monitoring device or neurosensor 53. Such a sensor 53 could consist of a comparator with a threshold value serving as a condition for the presence of an arrhythmia. If sensed nervous activity meets the condition, the comparator issues an arrhythmia-indicating output signal. So normal sinus rhythm and abnormal conditions in the heart, the latter possibly being bradycardia, hemodynamically unstable tachycardia and ventricular fibrillation requiring treatment, as well as nerve (sympathetic) signal activity indicating that the above conditions are established or impending, are detected in the detection block 5.

Data from the detection block 5 are sent to the control unit 13 which, depending on the data, orders the requisite therapy, such as tachycardia-terminating heart stimulation, and also sends a command signal to at least one of the blocks 7, 9 and 11, i.e. in the exemplified tachycardia-stimulating stimulation of the pacemaker block 7.

In addition to the nerve-heart defibrillator block 9 and parts of the detection device 5 (the neurosensor 53 in particular), the hitherto described parts and functions are conventional in nature, as noted above. So they will henceforth only be considered to the extent they relate to the nerve-heart stimulator block 9, which will now be described, and the neurosensor 53, to be described subsequently, in the following review.

Block 9 consists of a current generator 91 for nerve stimulation and is capable of supplying nerve-activating pulsed current with a balanced average current level , e.g. with a frequency range of 20 to 50 Hz, a pulse amplitude of 0-9 V and a pulse width of 0.1-1 ms, from a nerve stimulator 910, in addition to nerve-blocking direct current/high-frequency current to be discussed subsequently. The block 9 further comprises a time control unit 92 which is capable of supplying control information to the current generator 91 on e.g. which activating and blocking pulses, pulse sequences and continuous output signals should be delivered via the electrode system 23 from the block 9 to the sympathetic nerve 26 and vagus nerve 27 respectively, and also when the pulses are to be emitted. It should also be noted that the pulses supplied from block 9 may additionally comprise other suitable forms of pulses, such as dual biphasic pulses and alternatingly biphasic pulses separated by a pulse interval. The current generator's 91 and the time control unit's 92 parameters are, like other parameters in the implant 1, programmable via the telemetry unit 17. Therapy supplied from the block 9 can be supplied, repeatedly if need be, over a period of time, e.g. 5 to 10 seconds, suitable to the therapy. The time control unit 92 is shown, merely for illustrative purposes, as a separate unit in the block 9. It can naturally be an integrated part of the current generator 91.

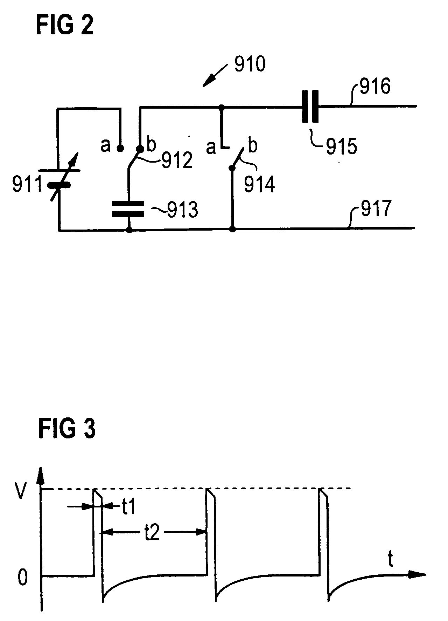

FIG. 2 shows an example of the nerve stimulator 910, which emits pulsed current for activating nerve stimulation, in the current generator 91. A voltage source 911 with a variable voltage V is connectable, via a switch 912, to a capacitor 913 with capacitance C. The capacitor 913 is also connectable, via the switch 912, to a capacitor 915, also with capacitance C. The capacitor 915 is connectable, via a switch 914, to an electrode output terminal 917. The nerve stimulator 910 can assume two states, a first state when the two switches 912, 914, both of which controlled in parallel by the time control unit 92, assume the position marked a in FIG. 2 and a second state when the two switches 912, 914 assume the position marked b. In the second state, the capacitors 913, 915 are connected in series, whereupon the capacitor 913, which is charged to voltage V from the voltage source 911, is discharged via the capacitor 915 and the electrode output terminals 916, 917. In the first state, the capacitor 913 is connected to the voltage source 911 by the switch 912, whereupon the capacitor 913 is recharged to the voltage V. In the first state, the capacitor 915 is also discharged via the electrode output terminals 916, 917 and the patient. Control of events is exercised by the time control unit 92. The capacitance C for the capacitors 913, 915 could e.g. be 100 µF.

Examples of pulses emitted by the block 9 are provided in FIG. 3. FIG. 3 shows the output signal over time t between the electrode output terminals 916, 917 in FIG. 2. The pulse width t1 could be 0.5 ms, and the pulse interval t2 could be 50 ms (20 Hz) in moderate stimulation. In maximum stimulation, t2 is reduced to about 20 ms (50 Hz). The amplitude of the output voltage V is not affected as long as V is above a threshold for stimulation of all fibers in the nerve. The threshold is electrode-related and amounts to about 5 volts for the electrode used here and described below.

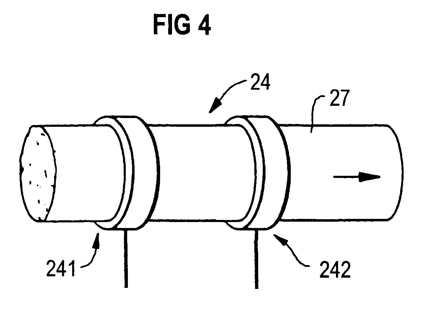

An electrode (to be described below for the vagus nerve in conjunction with FIG. 4) in the system 23 and electrode cable for the respective nerve to be stimulated can, as far as the electrode cable is concerned, consist of one or more flexible electrical conductors made of e.g. MP35, each conductor being enclosed in electrical insulation made of e.g. silicone rubber. Collective silicone rubber insulation on the conductors serves as the electrode cable's outer sheath. The electrode is devised for bipolar stimulation and has a first sub-electrode for the cathode and a second sub-electrode for the anode.

In constructive respects, the sub-electrodes can be devised as cuffs, rings, helices or the like with e.g. platinum, and other electrically conducting metals and/or polymers, as well as carbon fibres/meshes as electrode material in contact with the nerve and an electrically insulating and mechanically resilient sheath of silicone rubber around the electrode material, whereby the silicone rubber is pre-tensioned to some degree so the electrode, after implantation, retains mechanical and electrical contact with the nerve. The electrode can also be provided with suturing appliances and a device for mechanically relieving the load on the sub-electrodes, e.g. silicone rubber anchoring around the nerve with tensile relief for the sub-electrodes' conductors. The electrode could also be anchored, then with a constructively adapted design, in a blood vessel, preferably a venous vessel, immediately adjacent to the nerve.

A construction which is similar in all essential respects to the construction described for the stimulation electrodes can also be used for the sensor electrode employed for sensing heart-related activity in the sympathetic nerve. The vagus nerve could also be used, but the description relates to the sympathetic nerve as an example, whereby the nerve signals are sent to the nerve signal monitoring device 53 in the detection block 5. The sensor electrode for the sympathetic nerve 26 can also be the same as the stimulation electrode for the sympathetic nerve 26.

FIG. 4 shows an example of the construction principles for a cylindrical nerve electrode used here and applicable to a nerve. The FIG. shows the vagus nerve 27 and an electrode 24, consisting of a sub-electrode 241 arranged distal to the heart and a sub-electrode 242 arranged proximal to the heart 25, arranged thereon. The arrow in the FIG. 4 points towards the heart 25. The sub-electrodes 241 and 242 are for activating stimulation and connected via conductors in the system 23 (FIG. 1) to the plus output terminal 916 and the minus output terminal 917 respectively of the nerve stimulator 910. The result is that the main direction of nerve impulses is towards the heart 25.

As previously noted, the current generator 91 can also emit a current for blocking the sympathetic nerve 26, in addition to emitting the described pulses from the nerve stimulator 910 for activating the vagus nerve 27. One such blocking current can be achieved by additionally arranging in the current generator 91 a pole-reversed nerve stimulator 910, described in FIG. 2, so the sub-electrode 242 becomes positive and so the sub-electrode 24 negative. Here, the frequency of the emitted blocking pulses should range from 200 to 500 Hz so the action potential in the nerve never has time to drop. Another way to achieve a nerve blockage is to provide the current generator 91 with a direct current generator for emitting a direct current which can be applied to the sympathetic nerve 26 as a direct current from the plus pole of the direct current generator for e.g. a few seconds. Also, instead of a direct current a square wave provided by a square wave generator can be employed.

Stimulation and any sensor electrodes for the sympathetic nerve and the vagus nerve are preferably implanted in the patient's neck area. For the vagus nerve 27, the preferred implantation site is in the neck area by or near the right middle portion of the external carotid artery. For the sympathetic nerve, the preferred implantation site, as regards stimulation, is the ganglion stellatum, whereby an electrode adapted to use with this thickened part of the nerve is employed.

The nerve-heart defibrillator described here and comprised in block 9 therefore achieves defibrillation of the heart 25 by delivering an activating current to the vagus nerve 27 and a blocking current to the sympathetic nerve 26 from the block 9 in response to one or more fibrillation conditions detected by the devices 51, 52 and 53 in the detection block 5. If the defibrillation persists, despite this treatment (which can be repeated if necessary) from the nerve-heart defibrillator block 9, the control unit 13 can order collaboration with other parts of the defibrillator implant 1 which are relevant to the persistent fibrillation condition, so one or more electrical defibrillation shocks are emitted by the block 11 for electrical defibrillation.

It should be noted that the nerve-heart stimulator 9 according to the invention in the defibrillator implant 1 is also capable of treating, as previously noted, impending but as yet unestablished fibrillation conditions (or other refractory tachyarrhythmia) by prophylactically applying an activating current to the vagus nerve 27 and a blocking current to the sympathetic nerve 26, as described above.

Here, the nerve signal monitoring device 53 contributes to improved monitoring of the detection block 5 as regards tachyarrhythmias. The device 53 is e.g. arranged to be able to observe changes in the signal patterns of the autonomic nervous system generated by e.g. myocardial ischemia, a condition which often precedes a tachyarrhythmia. When the signal patterns are registered with an electrode described here (FIG. 4) and these patterns are processed (e.g. compared to patterns in normal conditions), changes can be detected in good time before dangerous tachyarrhythmia becomes established.

One example of the course in treatment with the nerve-heart defibrillator 9, utilizing the neurosensor 53 and collaborating with other units in the defibrillator implant 1, is provided below.

As soon as the detector block 5 detects an impending fibrillation or some other dangerous, impending tachyrhythmia (e.g. a change in the activity of the autonomic nervous system), treatment from the block 9 can be started in the form of light activation of the vagus nerve 27 for 5 seconds. If the block 5 detects a return to a normal state for the heart 25, treatment is terminated. But if the detector 5 continues to detect an abnormal condition for the heart 25, treatment will continue, supplemented with blocking of the sympathetic nerve, preferably at the ganglion stellatum, for a few seconds. If heart activity drops below a given rate because of the current delivered to the vagus nerve and the sympathetic nerve, the pacemaker block 7 automatically begins stimulating the heart 25 in order to maintain or restore its sinus rhythm. Treatment is terminated if the detector 5 now shows that the heart 25 has returned to a normal state. If this is not the case, the electrical defibrillator block 11 can be activated in order to shock the heart 25 in the conventional way.

Even if the nerve-heart stimulator 9 has been described for an application which also comprises many other units in a conventional implant, the described application obviously only shows the nerve-heart defibrillator's 9 possibilities and shall not be interpreted as any restriction on its use. The nerve-heart stimulator 9 can also, in treatment of supraventricular arrhythmias, only comprise the parts which stimulate the vagus nerve. In the treatment of supraventricular arrhythmias, the nerve-heart stimulator does not necessarily have to be implanted in the patient's body. It can also be used extracorporeally, e.g. for temporary use with appropriately situated external and internal nerve electrodes.

## Claims

1. A device for supraventricular heart therapy, whereby the device contains an arrhythmia detector (5) for detecting supraventricular arrhythmia and a nerve stimulator (910) for emitting pulses, in response to said detection, to a physiological representative (27) of the parasympathetic nervous system via an electrode system (23), **characterized in** that the electrode system's comprises stimulation means (24) devised to be placeable in an extracardiac position in the neck area of the physiological representative (27) of the parasympathetic nervous system and for activating this nervous system in direct contact there with this representative (27) or via an adjacent blood vessel.

2. A device according to claim 1, **characterized in** that the stimulation unit (24) consists of an electrode (24) with two sub-electrodes (241, 242) arranged at a distance from each other along the physiological representative's (27) longitudinal direction.

3. A device according to claim 1 or 2, **characterized in** that the device comprises a pacemaker block (7) for emitting pacemaker pulses in the ventricle of the heart (25) in response to said detection.
